# EUROPÄISCHE PATENTSCHRIFT

(11) **EP 0 902 285 B1**
(45) Veröffentlichungstag und Bekanntmachung des Hinweises auf die Patenterteilung: **11.12.2002**
(21) Anmeldenummer: 98116397.5
(22) Anmeldetag: 29.08.1998
(51) Int. Cl.: G01N 33/44, G01N 3/02

(54) **Verfahren und Testplatte zur Qualitätsbeurteilung von thermoplastischem Kunststoffmaterial**
Method and test plate for quality evaluation of thermoplastic synthetic material
Procédé et plaque d'essais pour l'évaluation de la qualité d'un matériau synthétique thermoplastique

(30) Priorität: 10.09.1997 DE 19739599
(43) Veröffentlichungstag der Anmeldung: 17.03.1999
(73) Patentinhaber: GRUNDIG Aktiengesellschaft, 90471 Nürnberg (DE)
(72) Erfinder: Biegel, Horst, 90762 Fuerth (DE); Schwarz, Gerhard, 90762 Fuerth (DE); Schultheiss, Klaus, 90762 Fuerth (DE)

(56) Entgegenhaltungen:
- WO-A-92/03715
- DE-A- 2 500 039
- DE-A- 4 405 540
- DE-C- 4 225 025
- DE-C- 4 408 860

## Beschreibung

Die Erfindung betrifft ein Verfahren und eine Testplatte zur Qualitätsbeurteilung von thermoplastischem Kunststoffmaterial.

Bereits heute nehmen beispielsweise Hersteller von Geräten der Unterhaltungselektronik Altgeräte vom Kunden zurück, um eine umweltschonende Gerätestillegung zu ermöglichen. Diese zurückgenommenen Geräte, die aus einer Vielzahl verschiedener Materialien zusammengesetzt sind, werden fachgerecht zerlegt, so daß die verschiedenen Materialien voneinander getrennt werden können. Diese verschiedenen Materialien werden dann teilweise entsorgt und teilweise wiederverwertet. In diesem Zusammenhang ist es bereits bekannt, aus Kunststoffteilen alter Geräte aus der Unterhaltungsindustrie Downcycling-Applikationen wie zum Beispiel Kugelschreiber oder Parkbänke herzustellen, wobei zunächst aus den alten Kunststoffteilen Kunststoffmahlgut gewonnen und dieses dann zum gewünschten neuen Produkt weiterverarbeitet wird. Diese Art von Recycling zeichnet sich grundsätzlich dadurch aus, daß die Qualitäts- und Sicherheitsanforderungen, die bei der Wiederverwertung an das Kunststoffmaterial zu stellen sind, weit niedriger sind als bei der originären Wiederverwertung als Gehäuseteil, so daß die Auswahl des Kunststoffmaterials im allgemeinen keine großen Schwierigkeiten bereitet.

Soll hingegen eine originäre Wiederverwendung des Kunststoffmaterials als Gehäuseteil erfolgen, dann muß sichergestellt sein, daß die aus dem wiederverwendeten Kunststoffmaterial hergestellten Gehäuseteile die erforderlichen Qualitäts- und Sicherheitsanforderungen erfüllen. So muß das wiederzuverwendende Kunststoffmaterial frei sein von Verunreinigungen oder Vermischungen mit Fremdpolymeren und anderen Störstoffen, wie beispielsweise Holz und Metall. Weiterhin muß das aus dem wiederverwendeten Kunststoffmaterial hergestellte Endprodukt bestimmte Oberflächeneigenschaften und Eigenschaften in bezug auf die Entflammbarkeit aufweisen, usw.

Die Überprüfung dieser und weiterer Anforderungen erfolgt nach dem bekannten Stand der Technik, bei welchem Rohmaterial zur Herstellung des Endproduktes verwendet wird, erst durch Auswertung von Mustergeräten im Anschluß an den Bau einer Vorserie. Zeigt sich bei dieser Überprüfung, daß die gewünschten Anforderungen nicht erfüllt sind, muß der gesamte Fertigungsvorgang inklusive des verwendeten Materials überprüft und ggf. abgeändert werden, damit die notwendigen Qualitäts- und Sicherheitsmerkmale erreicht werden können. Dieses Vorgehen ist zeitaufwendig und teuer.

Ein Geräte-Recycling im Sinne einer originären Wiederverwertung von Kunststoffteilen ist in der Vergangenheit stets an dem zu hohen Analytikaufwand und den damit zu hohen Folgekosten gescheitert, die mit dieser originären Wiederverwertung verbunden waren. So muß in der Praxis das für eine derartige Wiederverwendung notwendige gebrauchte Kunststoffmaterial von einer Vielzahl von Lieferanten bezogen werden, um für die beabsichtigte Großserienherstellung die erforderliche Menge von gebrauchtem Kunststoffmaterial vorgegebener Art zur Verfügung zu haben. Zu diesen Sekundärt-Lieferanten gehören Firmen unterschiedlicher Größen, die dem Wiederverwerter zunächst "unbekanntes" Material in großer Menge in Form von Kunststoffmahlgut zur Verfügung stellen.

Zeigt sich dann im Rahmen einer Vorserienfertigung, daß die Kunststoffteile der hergestellten Geräte die an sie zu stellenden Qualitäts- und Sicherheitsanforderungen nicht erfüllen, dann liegen beim Wiederverwerter große Mengen unbrauchbaren Kunststoffmahlguts vor, die entweder entsorgt oder an den jeweiligen Lieferanten, sofern dieser überhaupt noch feststellbar ist, zurückgeschickt werden müssen. Das Nichterfüllen der gewünschten Qualitäts- und Sicherheitsanforderungen kann unter anderem darauf zurückzuführen sein, daß im Hause des Lieferanten keine Möglichkeit zu einer Überprüfung des Materials besteht oder auch darauf, daß im Hause des Lieferanten die Trennung des gewünschten Kunststoffmaterials aus den gebrauchten Geräten und/oder Produkten nicht mit der nötigen Sorgfalt oder aufgrund mangelnder Kenntnisse erfolgt.

Aus WO 92 03 715 ist ein Equipment zur Überprüfung von Kunststoffmaterial bekannt. Hierbei steht eine Überprüfung der mechanischen Eigenschaften eines Kunststoffes im Vordergrund. Es werden, anhand von zwei herzustellenden Kunststoffteilen mechanische Eigenschaften überprüft. So weist ein Formteil diverse Haken und ein zweites Formteil zu diesen Haken passende Ausformungen auf, an welchen die mechanischen Eigenschaften des Kunststoffes anhand dieser Verbindungen getestet werden können. Im Weiteren wird überprüft, ob eine Verbindung, insbesondere eine Verklebung der herzustellenden Kunststoffteile, möglich ist.

Aus DE-A-25 00 039 ist ein Prüfkörper zur Verhaltensprüfung von Kunststoffen bekannt. Die Erfindung bezieht sich auf einen plattenförmigen Probekörper zur Untersuchung der physikalischen Eigenschaften eines insbesondere für Spritzgießwerkzeuge geeigneten Kunststoffes.

Aus DE-A-44 08 860 ist ein weiterer Prüfkörper zur Bestimmung der mechanischen Eigenschaften von polymeren Werkstoffen offenbart.

Aus DE-A-42 25 025 ist ein Probekörper aus Kunststoff zur Prüfung des Kunststoffes auf Beständigkeit gegenüber bestimmten Chemikalien unter mechanischer Beanspruchung bekannt.

Ausgehend von diesem Stand der Technik liegt der Erfindung die Aufgabe zugrunde, einen Weg aufzuzeigen, wie die vorstehend beschriebenen Nachteile beseitigt werden können, so daß eine originäre Wiederverwertung von thermoplastischem Kunststoffmaterial vereinfacht und verbilligt wird.

Diese Aufgabe wird durch eine Testplatte mit dem in Anspruch 1 angegebenen Merkmalen gelöst. Vorteilhafte Ausgestaltungen und Weiterbildungen der Erfindung sind in den Ansprüchen 2 bis 5 angegeben. Die Ansprüche 6 und 7 betreffen eine Spritzgußform zur Herstellung der Testplatte und die Ansprüche 8 bis 9 betreffen ein Verfahren zur Herstellung einer derartigen Testplatte.

Die Vorteile der Erfindung bestehen insbesondere darin, daß der Wiederverwerter vom jeweiligen Lieferanten zunächst nur eine kleine Menge an Kunststoffmahlgut, z.B. maximal 5 bis 10 kg, welches aus gebrauchten Geräten separiert wurde, anfordern kann. Diese kleine Menge Kunststoffmahlgut wird im Hause des Wiederverwerters zur Herstellung einer charakteristischen Testplatte mittels einer Spritzgußmaschine verwendet. Anhand dieser Testplatte und der daraus gewonnenen Ergebnisse kann der Wiederverwerter durch eine analoge Gesamtbetrachtung erkennen, ob das gelieferte Kunststoffmahlgut die gewünschten Eigenschaften aufweist oder nicht.

Ausgehend von diesem Stand der Technik liegt der Erfindung die Aufgabe zugrunde, einen Weg aufzuzeigen, wie die vorstehend beschriebenen Nachteile beseitigt werden können, so daß eine originäre Wiederverwertung von thermoplastischem Kunststoffmaterial vereinfacht und verbilligt wird.

Diese Aufgabe wird durch ein Verfahren mit den im Anspruch 1 angegebenen Merkmalen gelöst. Vorteilhafte Ausgestaltungen und Weiterbildungen der Erfindung sind in den Ansprüchen 2 - 11 angegeben. Die Ansprüche 12 - 15 betreffen eine Testplatte zur Qualitätsbeurteilung von thermoplastischem Kunststoffmaterial und die Ansprüche 16 und 17 eine Spritzgußform zur Herstellung einer derartigen Testplatte.

Die Vorteile der Erfindung bestehen insbesondere darin, daß der Wiederverwerter vom jeweiligen Lieferanten zunächst nur eine kleine Menge an Kunststoffmahlgut, z.B. maximal 5 bis 10 kg, welches aus gebrauchten Geräten separiert wurde, anfordern kann. Diese kleine Menge Kunststoffmahlgut wird im Hause des Wiederverwerters zur Herstellung einer charakteristischen Testplatte mittels einer Spritzgußmaschine verwendet. Anhand dieser Testplatte und der daraus gewonnenen Ergebnisse kann der Wiederverwerter durch eine analoge Gesamtbetrachtung erkennen, ob das gelieferte Kunststoffmahlgut die gewünschten Eigenschaften aufweist oder nicht. Liefert diese Überprüfung ein positives Ergebnis, dann kann der Wiederverwerter vom Lieferanten die dort vorhandene Restmenge an Kunststoffmahlgut anfordern. Hat die Überprüfung hingegen nicht das gewünschte Ergebnis, dann verbleibt das restliche Kunststoffmahlgut beim Lieferanten. Auf diese Weise ist sichergestellt, daß im Hause des Wiederverwerters keine großen Mengen unbrauchbaren Kunststoffmahlguts zwischengelagert und vom Wiederverwerter entsorgt werden müssen. Weiterhin ist auf diese Weise sichergestellt, daß sogenannte "schwarze Schafe" unter den Lieferanten nicht Kasse machen können, ohne Material ausreichender Qualität geliefert zu haben und führt zusätzlich zu einer Reduktion unnötiger Transportkosten.

Weitere Vorteile der Erfindung ergeben sich aus der beispielhaften Erläuterung der Erfindung anhand der Figuren. Es zeigt
- FIG 1: eine Testplatte nach einem ersten Ausführungsbeispiel für die Erfindung und
- FIG 2: eine Testplatte nach einem zweiten Ausführungsbeispiel für die Erfindung.

Die Erfindung bietet beispielsweise einem Fernsehgerätehersteller die Möglichkeit, Fernsehgehäuse im Rahmen einer Großserienfertigung und im Sinne einer originären Wiederverwendung aus thermoplastischem Kunststoffmaterial herzustellen, welches aus Altgeräten abgetrennt wurde.

Da zu diesem Zweck große Mengen an derartigem gebrauchten Kunststoffmaterial, beispielsweise ABS-Material, notwendig sind, steht der Fernsehgerätehersteller mit einer Vielzahl von Lieferanten in Kontakt. Bei diesen Lieferanten kann es sich sowohl um andere Industriefirmen handeln, in deren Betrieb Produktionsrückläufe in Form des gewünschten ABS-Materials entstehen, oder auch um Entsorgungsfirmen, die sich auf die Einsammlung und Verwertung von Altgeräten spezialisiert haben. Diese Firmen stellen nach den Vorgaben des Fernsehgeräteherstellers große Mengen des benötigten ABS-Materials, beispielsweise als komplette Teile, zusammen und bieten dieses dem Fernsehgerätehersteller in Form von Kunststoffmahlgut an.

Da dieser darauf angewiesen ist, nur Ware zu bekommen, die die erforderlichen Qualitäts- und Sicherheitsanforderungen erfüllt, läßt er sich vom jeweiligen Lieferanten vorerst eine kleine Menge dieser Ware zukommen, beispielsweise 5 bis 10 kg. Diese kleine Menge an Kunststoffmahlgut wird dazu verwendet, um im Hause des Fernsehgeräteherstellers mittels einer Spritzgußmaschine und einer entsprechend ausgestalteten Spritzgußform gegebenenfalls mehrere Testplatten herzustellen, aufgrund derer eine Überprüfung durchgeführt wird, ob die gewünschten Eigenschaften des thermoplastischen Materials vorliegen oder nicht.

Als besonders vorteilhaft hat es sich erwiesen das Kunststoffmahlgut vor dem Herstellen der Testplatte schwarz einzufärben. Die Einfärbung erfolgt mittels sogenannter Ruß- oder Farbbatches. Für jedes Kunststoffmaterial ist jeweils ein entsprechendes Farbbatch nötig. Durch die Schwarzeinfärbung werden die nachfolgend beschriebenen Erscheinungen besonders deutlich sichtbar.

Im weiteren kann anhand von Testplatten Aufschluß über entstaubtes Kunststoffmahlgut bzgl. dessen Homogenisierung in einer Plastifiziereinheit einer Spritzgußmaschinen gewonnen werden.

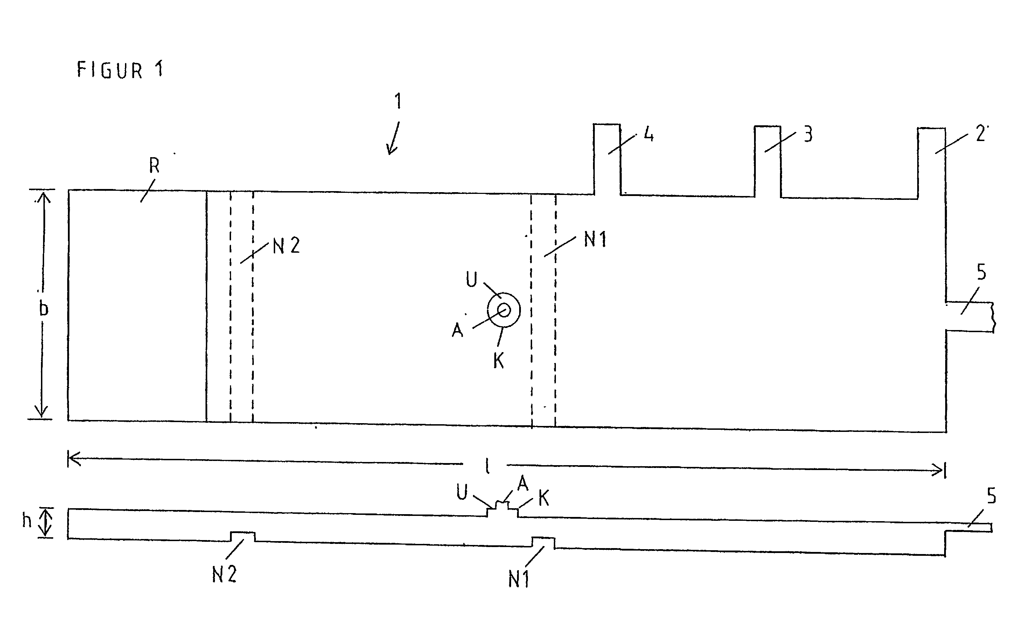

Ein erstes Ausführungsbeispiel für eine derartige Testplatte ist in der FIG 1 gezeigt. Die gezeigte Testplatte besteht aus einem rechteckigen Grundkörper 1, dessen aus der unteren Darstellung von FIG 1 ersichtliche Höhe h wesentlich kleiner ist als Länge I und Breite b. Die Testplatte weist zwei an ihrer Unterseite vorgesehene Nuten N1 und N2 auf, die in Figur gestrichelt angedeutet sind. Diese beiden Nuten N1 und N2 weisen von der Angußstelle A, d.h. von der Stelle, an der beim Einspritzvorgang das in die Spritzgußform eingespritzte geschmolzene Kunststoffmaterial ausgeht, verschiedene Abstände auf. Dadurch ist die Kunststoffmasse unterschiedlicher Scherung ausgesetzt.
Weiterhin weist die gezeigte Testplatte einen erodierten Oberflächenbereich R auf, der beim Spritzgießvorgang aufgrund einer entsprechend geformten Spritzgußform erzeugt wurde. Dies sorgt für zusätzliche Scherung im Angußbereich.

Femer ist eine sich kreisförmig um den Angußbereich A erstreckende, die Testplatte nach oben überstehende Überhöhung U vorgesehen, die mit einer umlaufenden Kante K versehen ist.

Schließlich weist die in der FIG 1 gezeigt Testplatte drei sich an einer der Längsseiten des Grundkörpers 1 nach außen erstreckende, rechteckige Verlängerungsstücke 2, 3, 4 und eine Überlaufbohne 5 auf.

Zur Herstellung dieser Testplatte mittels einer Spritzgußmaschine wird zunächst das angelieferte Kunststoffmahlgut mehrere Stunden lang bei vorzugsweise 80°C in einem Trockenofen getrocknet und mit Farbbatch homogenisiert. Danach wird das getrocknete Kunststoffmahlgut bei einem vorgegebenen ersten Temperaturprofil von beispielsweise 240°C plastifiziert. Nach diesem Plastifiziervorgang erfolgt ein Einspritzen des Materials in die Spritzgußform an einer Stelle, die der in der FIG 1 gezeigten Angußstelle A entspricht. Dieses Einspritzen erfolgt mit einer vorgegebenen ersten Materialeinspritzgeschwindigkeit von beispielsweise 100 mm/s.

Kommt es bei diesem Spritzvorgang zu Schlierenbildungen an der Testplatte, werden weitere Testplatten hergestellt, wobei andere Temperaturprofile wie beispielsweise 220°C und 260°C, und/oder andere, insbesondere verringerte, Materialeinspritzgeschwindigkeiten, wie beispielsweise 90 mm/s, 80 mm/s, bis hinab zu 10mm/s, usw. verwendet werden.

Eine Qualitätsbeurteilung erfolgt durch eine, insbesondere analoge, Auswertung der Eigenschaften der erzeugten Testplatte(n), sowie eine qualitative Abschätzung des Störbildes auf Ursachen bzw. Gesamtzusammenhänge. Zunächst wird überprüft, ob die Testplatte komplett gespritzt worden ist, oder ob Teile der Testplatte nicht vorhanden sind, d.h. der Kunststoff beim Spritzvorgang die gesamte Spritzform nicht gefüllt hat.
Im weiteren wird qualitativ der Schmelzindex des thermoplastischen Materials durch eine Messung der Länge der Überlaufbohne 5 festgestellt, wobei diese Fließfähigkeit bei den verschiedenen Temperaturen bzw. Einspritzgeschwindigkeiten in der Regel unterschiedlich ist. Weiterhin wird überprüft, ob bei den jeweils vorliegenden Bedingungen auf der Testplatte eine Schlierenbildung erfolgt ist oder nicht. Je nachdem ob bei bestimmten Bedingungen eine Schlierenbildung erfolgt ist oder nicht und auch aus der Form der gebildeten Schlieren können Rückschlüsse auf die Tauglichkeit des vorliegenden Kunststoffmahlguts zur originären Wiederverwertung gezogen werden. So kann beispielsweise festgestellt werden, ob das vorhandene Material in gewünschter Weise homogenisierbar ist. Ist der qualitativ bewertete Schmelzindex bei bestimmten Bedingungen geringer als ein gewünschter Wert, dann wird aufgrund der dann nicht vollständig gespritzten Testplatte darauf geschlossen, daß das Material untauglich ist.

Im weiteren kann anhand einer Schlierenbildung auf der Testplatte erkannt werden, ob das Kunststoffmaterial mit einem zu hohen Feuchtigkeitsgehalt verarbeitet wurde. Dies kann Rückschlüsse auf eine schlechte Materialtrocknung zulassen.

Eine Auswertung im Hinblick auf eine Schlierenbildung ermöglicht es weiterhin, Rückschlüsse auf die Scherempfindlichkeit des vorliegenden Kunststoffmaterials zu ziehen. In diesem Zusammenhang sind Schlieren von Bedeutung, die sich an den Rändern der Nuten N1 und N2 sowie im Bereich der Überhöhung U in Fließrichtung des Kunststoffmaterials ergeben.

Mittels der genannten Testplatte erfolgt auch eine Überprüfung im Hinblick auf das Vorliegen von unerwünschten Fremdstoffen im Kunststoffmaterial. Störstoffe können in Form von anderen Kunststoffen, Wasser bzw. Feuchtigkeit im Kunststoffmahlgut, oder von echtem Fremdmaterial, wie z.B. Papier, Holz, Metall usw. vorliegen. Bei vorhandenem Wasser oder zu großer Feuchtigkeit im Kunststoffmahlgut entstehen typische Feuteschlieren auf der Testplatte.

Anhand des erodierten oder geätzten Oberflächenbereiches R erfolgt ein subjektiver Farbvergleich in Form einer Oberflächenüberprüfung, um die Oberflächeneigenschaften des vorliegenden Kunststoffmahlguts für den Fall überprüfen zu können, daß die Oberfläche des späteren Gehäuses nicht mehr lackiert werden soll. Der Oberflächenbereich R ist in seinen Abmessungen auch an die optischen Meßeinrichtungen zur Farbmessung angepaßt, sodaß eine objektive Farbmessung durchgeführt werden kann.

Anhand eines weiteren Oberflächenbereiches der Testplatte, der hochglanzpoliert ist, kann eine Glanzmessung und ein subjektiver Farbvergleich in Form einer Oberflächenüberprüfung erfolgen, um die Oberflächeneigenschaften des vorliegenden Kunststoffmahlguts für den Fall überprüfen zu können, daß die Oberfläche des späteren Gehäuses nicht mehr lackiert werden soll. Dieser Oberflächenbereich ist ebenfalls in seinen Abmessungen an die optischen Meßeinrichtungen zur Glanzmessung angepaßt.

Beide Oberflächenbereiche sind plan ausgestaltet, damit die Meßköpfe der Meßgeräte eben aufliegen.

Die Verlängerungstücke 2, 3 und 4, die senkrecht zur Längsrichtung der Testplatte gerichtet sind, dienen ebenso wie weitere rechteckförmige Streifen, die der Testplatte an anderen Stellen entnommen und parallel zur Längsrichtung der Testplatte gerichtet sind, als Prüfstäbe zur Durchführung einer Flammprüfung, insbesondere nach den Normen EN 60065, VDE 0860 oder UL94/IEC707. Diese Prüfstäbe weisen jeweils gleiche Abmessungen auf und sind Bereichen der Testplatte entnommen, die unterschiedliche Entfernungen von der Angußstelle A haben, und somit eine unterschiedliche Orientierung der Polymerketten voliegt.

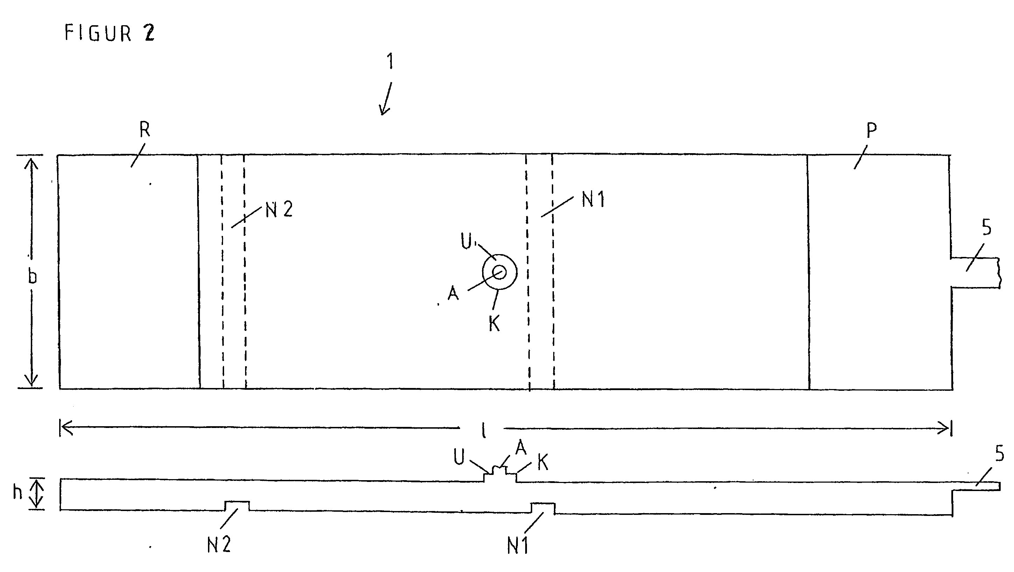

Die FIG 2 zeigt eine Testplatte nach einem zweiten Ausführungsbeispiel für die Erfindung. Die in der FIG 2 gezeigte Testplatte unterscheidet sich von der in der FIG 1 gezeigten im wesentlichen dadurch, daß sie keine an einer Längsseite seitlich nach außen überstehende Verlängerungsstücke aufweist. Statt dessen weist diese Testplatte neben einem erodierten Oberflächenbereich R auch einen hochglanzpolierten Oberflächenbereich P auf, mittels welchem eine Bestimmung des Glanzgrades und eine Farbmessung der Oberfläche erfolgen kann. Weiterhin werden bei diesem Ausführungsbeispiel die für die Flammprüfung benötigten Prüfstäbe sämtlich dem rechteckförmigen Grundkörper der Testplatte entnommen, wobei diese Entnahme am Ende des Prüfvorganges durch ein Herausschneiden der Prüfstäbe aus der Testplatte erfolgt.

Im weiteren ist vorgesehen die gesamte Testplatte in einer Klimakammer zu lagern. Hierdurch kann an der Testplatte festgestellt werden, ob in der Testplatte, und folglich im zu prüfenden thermoplastischen Kunststoffmaterial, Hilfsstoffe oder Additive vorhanden sind, die aus der Testplatte migrieren, sprich auswandern. Bei Vorliegen einer Migration kommt es zu Störungen bei einer späteren Lackierung. Dies kann im voraus erkannt werden.

Die Erfindung bietet nach alledem den Vorteil, daß das Vorliegen bzw. Fehlen aller für einen bestimmten Verwendungszweck notwendigen Eigenschaften von thermoplastischem Kunststoffmaterial anhand einer kleinen Menge dieses Materials durch Herstellung einer oder mehrerer Testplatten mittels einer Spritzgußmaschine ermittelt werden kann, wobei diese Ermittlung sowohl durch eine Inaugenscheinnahme der Testplatte als auch eine maschinelle Auswertung der Testplatte erfolgen kann. Die Spritzgußmaschine weist eine Spritzgußform auf, deren Innenbereich an die Form der Testplatte angepaßt ist. In vorteilhafter Weise ist die Spritzgußform derart höhenverstellbar, daß die Höhe bzw. Dicke einer mittels der Spritzgußform hergestellten Testplatte und auch die Tiefe der Nuten N1, N2 wählbar ist.
Ein weiterer Vorteil der Erfindung besteht darin, daß die genannte Spritzgußmaschine und die genannte Spritzgußform zur Überprüfung einer Vielzahl verschiedener Materialproben verwendet werden kann.

## Patentansprüche

1. Testplatte zur Qualitätsbeurteilung von thermoplastischem Kunststoffmaterial, welche im Spritzgußverfahren hergestellt wird und einen rechteckförmigen Grundkörper aufweist,
**dadurch gekennzeichnet, daß**
die Höhe des rechteckförmigen Grundkörper wesentlich kleiner als dessen Länge und Breite ist, die Testplatte einen Anguß (A) in zentraler Position, eine Überlaufbohne (5) und einen erodierten Oberflächenbereich (R) aufweist.

2. Testplatte nach Anspruch 1,
**dadurch gekennzeichnet, daß**
der Anguß (A) senkrecht auf Testplatte vorgesehen ist und/oder der Anguß (A) als Punktanguß ausgeführt ist.

3. Testplatte nach Anspruch 1 oder 2,
**dadurch gekennzeichnet, daß**
der Grundkörper der Testplatte mindestens zwei Nuten (N1, N2) aufweist, welche verschiedene Abstände vom Anguß (A) haben.

4. Testplatte nach Anspruch einem der Ansprüche 1 bis 3,
**dadurch gekennzeichnet, daß**
die Testplatte mehrere sich an einer Längsseite des Grundkörpers nach außen erstreckende rechteckförmige Verlängerungsstücke aufweist.

5. Testplatte nach einem der Ansprüche 1 bis 4,
**dadurch gekennzeichnet, daß**
die Testplatte eine sich kreisförmig um den Angußbereich erstreckende, die Testplatte nach oben überstehende Überhöhung aufweist, die mit einer umlaufenden Kante versehen ist, die Testplatte eine Hochglanzfläche (P) und eine aufgeraute Fläche aufweist.

6. Spritzgußform zur Herstellung einer Testplatte mit den Merkmalen eines oder mehrerer der Ansprüche 1 bis 5 wobei in die Spritzgußform ein erodierter Oberflächenbereich durch einen Ätzvorgang oder eine Funkenerosion eingebracht ist.

7. Spritzgußform nach Anspruch 6,
**dadurch gekennzeichnet, daß**
die Spritzgußform derart höhenverstellbar ist, daß die Höhe einer mittels der Spritzgußform hergestellten Testplatte wählbar ist.

8. Verfahren zur Qualitätsbeurteilung von thermoplastischem Kunststoffmaterial
**dadurch gekennzeichnet, daß**
aus dem thermoplastischen Kunststoffmaterial unter Verwendung einer Spritzgußmaschine eine Testplatte nach einem der Ansprüche 1 bis 5 hergestellt wird und die Qualitätsbeurteilung durch eine Auswertung der Eigenschaften der Testplatte erfolgt.

9. Verfahren nach Anspruch 8,
**dadurch gekennzeichnet, daß**
die Auswertung als eine analoge Auswertung vorgenommen wird.

10. Verfahren nach Anspruch 8 oder 9,
**dadurch gekennzeichnet, daß**
zur Quatitätsbeurteilung eine Messung der Fließlänge an einer sogenannten Überlaufbohne des auf eine vorgegebene Temperatur erhitzten thermoplastischen Materials bei vorgegebener Einspritzgeschwindigkeit vorgenommen wird.

11. Verfahren nach einem der Ansprüche 8 bis 10,
**dadurch gekennzeichnet, daß**
zur Qualitätsbeurteilung eine Auswertung im Hinblick auf das Vorliegen von unerwünschten Störstoffen, sowie Verunreinigungen jeglicher Art, insbesondere Papier, Wasser und/oder Feuchtigkeit im Kunststoffmahlgut, Metall, Lacke, usw., erfolgt.

12. Verfahren nach einem der Ansprüche 8 bis 11,
**dadurch gekennzeichnet, daß**
zur Qualitätsbeurteilung eine Auswertung des Schlierenbildes auf der Testplatte erfolgt, wobei die Testplatte bei einem festgelegten Temperaturprofil bei vorgegebener Materialeinspritzgeschwindigkeit erzeugt wird.

13. Verfahren nach einem der Ansprüche 8 bis 12,
**dadurch gekennzeichnet, daß**
zur Qualitätsbeurteilung eine Auswertung des Schlierenbildes aufgrund von Scherung des auf eine vorgegebene Temperatur erhitzten thermoplastischen Materials bei vorgegebener Einspritzgeschwindigkeit erfolgt.

14. Verfahren nach einem der Ansprüche 8 bis 13,
**dadurch gekennzeichnet, daß**
zur Qualitätsbeurteilung eine Auswertung der Oberflächeneigenschaften des auf eine vorgegebene Temperatur erhitzten thermoplastischen Materials bei vorgegebener Einspritzgeschwindigkeit, insbesondere auf Migration, Grad der Einfärbung und Farbmessungen, erfolgt.

15. Verfahren nach einem der Ansprüche 8 bis 14,
**dadurch gekennzeichnet, daß**
zur Qualitätsbeurteilung eine Flammprüfung erfolgt und die Flammprüfung anhand mehrerer der Testplatte entnommener Prüfstäbe erfolgt, die jeweils gleiche Abmessungen aufweisen und Bereichen der Testplatte entnommen sind, die unterschiedliche Entfernungen und Ausrichtungen bezüglich der Angußstelle der verwendeten Gußform aufweisen.

16. Verfahren nach einem der Ansprüche 8 bis 15,
**dadurch gekennzeichnet, daß**
die Qualitätsbeurteilung bei verschiedenen vorgegebenen Temperaturen erfolgt und die Testplatten mit verschiedenen vorgegebenen Einspritzgeschwindigkeiten hergestellt sind.

17. Verfahren nach einem der Ansprüche 8 bis 16,
**dadurch gekennzeichnet, daß**
das Verfahren zur Qualitätsbeurteilung von thermoplastischem Kunststoffmaterial vorgesehen ist, welches aus Kunststoffmahlgut besteht, das aus thermoplastisches Kunststoffmaterial aufweisenden Altgeräten oder Gegenständen gewonnen wurde.

18. Verfahren nach einem der Ansprüche 8 bis 17,
**dadurch gekennzeichnet, daß**
anhand von Testplatten Aufschluß über entstaubtes Kunststoffmahlgut bzgl. dessen Homogenisierung in einer Plastifiziereinheit einer Spritzgußmaschinen gewonnen wird.

19. Verfahren nach einem der Ansprüche 8 bis 18,
**dadurch gekennzeichnet, daß**
das verfahren zur Qualitätsbeurteilung von synthetisch neu erzeugtem thermoplastischem Kunststoffmaterial vorgesehen ist.

## Claims

1. Test plate for assessing the quality of thermoplastic synthetic material that is produced by the injection-moulding method and has a rectangular basic body, **characterized in that** the height of the rectangular basic body is substantially less than its length and width, and the test plate has a gate stub (A) in a central position, an overflow runner (5) and an eroded surface region (R).

2. Test plate according to Claim 1, **characterized in that** the gate stub (A) is provided perpendicularly to the test plate and/or the gate stub (A) is designed as point gate stub.

3. Test plate according to Claim 1 or 2, **characterized in that** the basic body of the test plate has at least two grooves (N1, N2) that are at different distances from the gate stub (A).

4. Test plate according to one of Claims 1 to 3, **characterized in that** the test plate has a plurality of rectangular elongation pieces extending outwards on a longitudinal side of the basic body.

5. Test plate according to one of Claims 1 to 4, **characterized in that** the test plate has an elevation that extends circularly around the gate region and projects upwards above the test plate and that is provided with a circumferential edge and the test plate has a high-gloss area (P) and a roughened area.

6. Injection mould for producing a test plate having the features of one or more of Claims 1 to 5, wherein an eroded surface region is introduced into the injection mould by an etching process or a spark erosion.

7. Injection mould according to Claim 6, **characterized in that** the injection mould can be adjusted in height in such a way that the height of a test plate produced by means of the injection mould is selectable.

8. Method of assessing the quality of thermoplastic synthetic material, **characterized in that** a test plate according to one of Claims 1 to 5 is produced from the thermoplastic synthetic material using an injection-moulding machine and the quality is assessed by an evaluation of the properties of the test plate.

9. Method according to Claim 8, **characterized in that** the evaluation is performed as an analog evaluation.

10. Method according to Claim 8 or 9, **characterized in that**, for the purpose of assessing quality, the flow length at a so-called overflow runner of the thermoplastic material heated to a predetermined temperature is measured at a predetermined injection rate.

11. Method according to one of Claims 8 to 10, **characterized in that**, for the purpose of assessing quality, an evaluation is performed with respect to the presence of undesirable contaminants, and also impurities of any type, in particular paper, water and/or moisture in the synthetic ground stock, metal, lacquers, etc.

12. Method according to one of Claims 8 to 11, **characterized in that**, for the purpose of assessing quality, the streak pattern on the test plate is evaluated, wherein the test plate is produced with a fixed temperature profile at a predetermined material injection rate.

13. Method according to one of Claims 8 to 12, **characterized in that**, for the purpose of assessing quality, the streak pattern is assessed on the basis of shearing of the thermoplastic material heated to a predetermined temperature at a predetermined injection rate.

14. Method according to one of Claims 8 to 13, **characterized in that**, for the purpose of assessing quality, the surface properties of the thermoplastic material heated to a predetermined temperature are evaluated at a predetermined injection rate, in particular for migration, degree of coloration and colour measurements.

15. Method according to one of Claims 8 to 14, **characterized in that**, for the purpose of assessing quality, a flame test is performed and the flame test is performed on the basis of a plurality of test rods that are taken from the test plate and that each have the same dimensions and are taken from regions of the test plate that are at different distances and have different alignments with respect to the injection point of the mould used.

16. Method according to one of Claims 8 to 15, **characterized in that** the quality is assessed at different predetermined temperatures and the test plates are produced with different predetermined injection rates.

17. Method according to one of Claims 8 to 16, **characterized in that** the method is provided for assessing the quality of thermoplastic synthetic material that is composed of synthetic ground stock obtained from scrap appliances or objects comprising thermoplastic synthetic material.

18. Method according to one of Claims 8 to 17, **characterized in that** information is obtained about dedusted synthetic ground stock with respect to its homogenization in a plasticator of an injection-moulding machine on the basis of test plates.

19. Method according to one of Claims 8 to 18, **characterized in that** the method is provided for assessing the quality of synthetic, newly produced thermoplastic synthetic material.

## Revendications

1. Plaque test pour évaluer la qualité de matériau plastique thermoplastique qui sera fabriquée en processus de moulage par injection et qui présente un corps de base rectangulaire,
**se caractérisant par le fait que**
la hauteur du corps de base rectangulaire est beaucoup plus faible que sa largeur et longueur, la plaque test présente un culot (A) en position centrale, un goujon déversoir (5) et une zone de surface érodée (R).

2. Plaque test selon la revendication 1,
**se caractérisant par le fait que**
le culot (A) est prévu à la perpendiculaire de la plaque test et/ou le culot (A) a la forme d'une injection capillaire.

3. Plaque test selon la revendication 1 ou 2,
**se caractérisant par le fait que**
le corps de base de la plaque test présente au moins deux rainures (N1, N2) qui sont placées à différentes distances du culot (A).

4. Plaque test selon l'une des revendications 1 à 3,
**se caractérisant par le fait que**
la plaque test présente plusieurs rallonges rectangulaires s'étendant vers l'extérieur sur un côté latéral du corps de base.

5. Plaque test selon l'une des revendications 1 à 4,
**se caractérisant par le fait que**
la plaque test présente une surélévation dépassant par le haut, s'étendant en forme de cercle autour de la zone du culot, qui est munie d'un bord sur tout le pourtour, la pâque test présente une surface polie miroir (P) et une surface rugueuse.

6. Moule de coulage pour injection pour la fabrication d'une plaque test avec les caractéristiques de l'une ou de plusieurs revendications 1 à 5, une zone de surface érodée étant intégrée dans le moule par corrosion ou par électroérosion.

7. Moule de coulage pour injection selon la revendication 6,
**se caractérisant par le fait que**
le moule de coulage est réglable en hauteur de sorte que l'on pourra choisir la hauteur de la plaque test fabriquée au moyen du moule de coulage.

8. Procédé d'évaluation de la qualité de matériau plastique thermoplastique
**se caractérisant par le fait que**
une plaque test selon l'une des revendications 1 à 5 sera fabriquée à partir du matériau plastique thermoplastique en employant une presse à injection et
que l'évaluation de la qualité aura lieu par le biais de l'évaluation des propriétés de la plaque test.

9. Procédé selon la revendication 8,
**se caractérisant par le fait que**
l'évaluation sera faite sous forme d'évaluation analogique.

10. Procédé selon la revendication 8 ou 9,
**se caractérisant par le fait que**
pour l'évaluation de la qualité une mesure de la longueur de fluidité sera faite sur un dit goujon déversoir du matériau thermoplastique chauffé à une température prédéterminée pour une vitesse d'injection prédéterminée.

11. Procédé selon la revendication 8 à 10,
**se caractérisant par le fait que**
pour l'évaluation de la qualité, une évaluation de la présence de substances parasites indésirables ainsi que d'impuretés en tous genres, en particulier de papier, d'eau et/ou d'humidité dans le matériau plastique moulu, de métal, de laque etc. sera faite.

12. Procédé selon la revendication 8 à 11,
**se caractérisant par le fait que**
pour l'évaluation de la qualité, une évaluation de l'image de stries sur la plaque test sera faite, la plaque test étant produite à un profil de température fixé pour une vitesse d'injection du matériau prédéterminée.

13. Procédé selon la revendication 8 à 12,
**se caractérisant par le fait que**
pour l'évaluation de la qualité, une évaluation de l'image de stries sera faite sur la bàse du cisaillement du matériau thermoplastique chauffé à une température prédéterminée pour une vitesse d'injection prédéterminée.

14. Procédé selon la revendication 8 à 13,
**se caractérisant par le fait que**
pour l'évaluation de la qualité, une évaluation des propriétés de surface du matériau thermoplastique chauffé à une température prédéterminée pour une vitesse d'injection prédéterminée, sera faite en particulier en considération de la migration, du degré de coloration et des mesures chromatométriques.

15. Procédé selon la revendication 8 à 14,
**se caractérisant par le fait que**
pour l'évaluation de la qualité, un test à la flamme sera fait et ce test à la flamme se fera à l'aide de plusieurs joncs d'essai prélevés sur la plaque test, ces joncs présentent les mêmes dimensions et sont prélevés dans les zones de la plaque test qui se trouvent à des distances et des orientations différentes par rapport au culot du moule de coulage utilisé.

16. Procédé selon la revendication 8 à 15,
**se caractérisant par le fait que**
l'évaluation de la qualité se fera à différentes températures prédéterminées et les plaques tests seront fabriquées à différentes vitesses d'injection prédéterminées.

17. Procédé selon la revendication 8 à 16,
**se caractérisant par le fait que**
le procédé d'évaluation de la qualité prévu se fera sur un matériau plastique thermoplastique qui se compose de matériau plastique moulu qui aura été extrait d'appareils ou d'objets usagés contenant du matériau plastique thermoplastique.

18. Procédé selon la revendication 8 à 17,
**se caractérisant par le fait que**
à l'aide des plaques tests, l'on pourra obtenir des renseignements sur le matériau plastique moulu dépoussiéré resp. sur son homogénéisation dans une unité de plastification d'une presse à injection.

19. Procédé selon la revendication 8 à 18,
**se caractérisant par le fait que**
le procédé d'évaluation de la qualité prévu se fera sur un matériau plastique thermoplastique synthétique, de fabrication neuve.
